# EUROPEAN PATENT APPLICATION

(11) **EP 4 814 979 A1**
(43) Date of publication of application: **30.09.2026**
(21) Application number: 24898193.8
(22) Date of filing: 29.11.2024
(51) Int. Cl.: G16H 40/60, G16H 50/20, G16H 10/60, G16H 20/00, G16H 40/40, A61B 5/00, G16H 40/20, G16H 80/00, A45D 19/02, A45D 19/00

(54) **SKINCARE MANAGEMENT PLATFORM BASED ON CARTRIDGE-REPLACEABLE SKINCARE DEVICE, AND SKINCARE MANAGEMENT METHOD**

(30) Priority: 30.11.2023 KR 20230170874
(71) Applicant: EnterTake Co., Ltd., Seoul 04626 (KR)
(72) Inventor: OH, Seong Min, Yongin-si, Gyeonggi-do 16803 (KR)
(74) Representative: Sander, Rolf
(86) International application number: PCT/KR2024/019253
(87) International publication number: WO 2025/116599

(57) **Abstract**

The present disclosure discloses a skincare management platform including: a user system including a skincare device on which a skincare product replaceable in a cartridge form is mountable, and a user terminal configured to collect skin condition information regarding a skin condition of a user using the skincare device; a skin condition diagnosis apparatus configured to receive the skin condition information from the user system, diagnose a skin condition of the user based on the skin condition information, and generate skin condition diagnosis information regarding the skin condition of the user according to a result of the diagnosis; an expert terminal configured to verify the skin condition diagnosis information and provide a skincare solution necessary for skincare of the user based on the skin condition diagnosis information; and a maker terminal configured to receive the skincare solution from the expert terminal and provide information regarding a skincare product and a customized skincare product to the user based on the skincare solution. According to the present disclosure, in addition to demand and supply of a skincare device, demand and supply of a skincare product in a cartridge form mounted on the skincare device, diagnosis of a skin condition necessary for skincare, and integrated management of skincare information provided by an expert and linked to the skincare product are possible.

## Description

### [Technical Field]

The present disclosure relates to a skincare management platform based on a cartridge-replaceable skincare device and a skincare management method, and more particularly, to a skincare management platform and method capable of diagnosing a condition of skin including a scalp by using a mobile terminal and providing information on skincare and a skincare product in a cartridge form, in a skincare device in which a cartridge of a hair dye and a hair nutrient or a cartridge of cosmetics can be replaced and used.

### [Background Art]

The content presented in this section only provides information serving as a background of the present disclosure and does not constitute prior art.

As a technology related to the present disclosure, a total hybrid skin and hair diagnosis method capable of one-stop diagnosis, disclosed in a Korean registered patent publication, includes a step of selecting an image, a step of counseling and diagnosing, a step of performing macroscopic diagnosis and detailed diagnosis, and a professional counseling step, but is possible through a magic mirror system when a consumer visits a store, and is distinguished from the configuration of the present disclosure in which a user at a remote place collects skin information by using a user terminal while using a skincare device, and diagnosis and solution provision are performed based on the collected skin information.

### [Disclosure]

### [Technical Problem]

One problem to be solved by the present disclosure is to provide a skincare management platform capable of providing a service in which diagnosis of a skin condition in skincare, provision of expert skincare information according to the diagnosis, sale and consumption of a skincare device according thereto, and sale and consumption of a skincare product in a cartridge form mounted on the skincare device are comprehensively linked, and a skincare management method using the same.

One problem to be solved by the present disclosure is to provide a skincare management platform in which a user directly collects skin information necessary for diagnosis of a skin condition of the user, diagnosis is performed at a remote place by using the collected skin information, and a solution can be provided to the user based on a diagnosis result.

One problem to be solved by the present disclosure is not limited to the problems mentioned above, and another problem not mentioned will be clearly understood by a person skilled in the art from the following description.

### [Technical Solution]

In order to achieve the above object, according to an embodiment based on the technical spirit of the present disclosure, disclosed is a skincare management platform including: a user system including a skincare device on which a skincare product replaceable in a cartridge form is mountable, and a user terminal configured to collect skin condition information regarding a skin condition of a user using the skincare device;

a skin condition diagnosis apparatus configured to receive the skin condition information from the user system, diagnose a skin condition of the user based on the skin condition information, generate skin condition diagnosis information regarding the skin condition of the user according to a result of the diagnosis, and provide information regarding a skincare product to the user based on skincare information; an expert terminal configured to verify the skin condition diagnosis information and provide the skincare information necessary for skincare of the user based on the skin condition diagnosis information; and a maker terminal configured to provide a customized skincare product to the user based on the skincare information and a selection of the user.

In addition, the skincare management platform may be configured such that the skincare device includes at least one of a scalp care device on which a hair dye or scalp care product replaceable in a cartridge form is mountable, and a face care device on which a face care product replaceable in a cartridge form is mountable.

In addition, the skincare management platform may be configured such that the skincare device includes a first cartridge containing a first content and a second cartridge containing a second content; a mixing unit configured to generate a mixture by mixing predetermined partial amounts of the first content and the second content with each other; and a discharge unit configured to discharge the mixture through a nozzle.

In addition, the skincare management platform may be configured such that the first content and the second content include a hair dyeing agent and an oxidizing agent forming the hair dye, a first liquid agent and a second liquid agent forming the scalp care product or the face care product, or a solid agent and a liquid agent forming the scalp care product or the face care product, and any one of the first liquid agent and the second liquid agent, or any one of the solid agent and the liquid agent includes a vitamin component.

In addition, the skincare management platform may be configured such that the skincare device further includes a display unit capable of displaying remaining amount information of the first content and the second content and replacement information of the first cartridge and the second cartridge.

In addition, the skincare management platform may be configured such that the display unit provides cleaning history information regarding cleaning of the mixing unit and the discharge unit to the user, and displays a message requesting cleaning to the user when cleaning of the mixing unit and the discharge unit is necessary.

In addition, the skincare management platform may be configured such that the skincare device further includes a cleaning cartridge replaceable with the first cartridge and the second cartridge, and operates in a cleaning mode in which residues of the first content or the second content accumulated in the mixing unit and the discharge unit are removed by using the cleaning cartridge.

In addition, the skincare management platform may be configured such that the skin condition diagnosis apparatus includes an image analysis unit configured to analyze an image included in the skin condition information received from the user system, a data collection unit configured to collect data regarding a skin condition by using a result of image analysis of the image analysis unit, and a skin condition diagnosis unit configured to diagnose a degree of hair loss by using the data regarding the skin condition.

In addition, the skincare management platform may be configured such that the skin condition diagnosis apparatus determines a degree of hair loss of the user by using the data regarding the skin condition and the following equation.$\begin{matrix}DoHL = α \left(\frac{{N}_{avr} - N}{{N}_{avr}}\times 100\right) + β \left(\frac{{T}_{avr} - T}{{T}_{avr}}\times 100\right) + γ \left(\frac{{NH}_{avr} - NH}{{NH}_{avr}}\times 100\right) \\ + δ \left(\frac{EH - {EH}_{avr}}{EH}\times 100\right)\end{matrix}$
*Nₐᵥᵣ*: average number of hairs per unit area (for n normal persons)
*Tₐᵥᵣ*: average hair thickness (m hairs measured for n normal persons)
*NHₐᵥᵣ*: average number of hairs per follicle per unit area (for n normal persons)
*EHₐᵥᵣ*: average number of empty follicles per unit area (for n normal persons)
*N*: average number of hairs per unit area of a measurement subject
*T*: average hair thickness of the measurement subject (n hairs measured)
*NH:* average number of hairs per follicle per unit area of the measurement subject
*EH:* average number of empty follicles per unit area of the measurement subject
α: hair number constant (value calculated by the company)
*β*: hair thickness constant (value calculated by the company)
*γ*: average hair number per follicle constant (value calculated by the company)
*δ*: empty follicle number constant (value calculated by the company)

In addition, the skincare management platform may be configured such that the skin condition diagnosis apparatus includes an artificial intelligence diagnosis model configured to diagnose a skin condition of the user by using the skin condition information based on an ability to diagnose a skin condition acquired by learning, and generate the skin condition diagnosis information based on a result of the diagnosis.

In addition, the skincare management platform may be configured such that the skin condition diagnosis apparatus further includes a first database storing scalp condition information according to at least one condition among age, sex, time, environmental condition, skin tone, hair length, disease, occupation, and scalp care history; and a second database storing degrees of scalp condition change of a plurality of users diagnosed and prescribed based on information in the first database, and the skin condition diagnosis unit diagnoses a condition of the user's scalp and hair based on the skin condition information, and diagnoses the user's scalp condition by comparing information and data stored in the first database and the second database with the skin condition information received from the user system.

In order to achieve the above object, according to an embodiment based on the technical spirit of the present disclosure, a method performed by a skincare management platform may include: collecting, by a user system included in the skincare management platform, skin condition information regarding a skin condition of a user using a skincare device on which a skincare product replaceable in a cartridge form is mountable; receiving the skin condition information from the user system, diagnosing a skin condition of the user based on the skin condition information, and generating skin condition diagnosis information regarding the skin condition of the user according to a result of the diagnosis; verifying the skin condition diagnosis information and providing skincare information necessary for skincare of the user based on the skin condition diagnosis information; and providing product information regarding a skincare product and a customized skincare product to the user based on the skincare information.

Specific matters of other embodiments are included in "Best Mode for Carrying Out the Invention" and the accompanying "Drawings."

Advantages and/or features of the present disclosure and methods of achieving the same will become apparent with reference to various embodiments described below in detail together with the accompanying drawings.

However, the present disclosure is not limited only to configurations of each embodiment disclosed below and may be implemented in various different forms, and each embodiment disclosed in the present specification is merely provided so that the disclosure of the present disclosure is complete and to fully inform a person having ordinary skill in the art to which the present disclosure pertains of the scope of the present disclosure, and it should be understood that the present disclosure is defined only by the scope of each claim of the claims.

### [Advantageous Effects]

According to the present disclosure, in addition to demand and supply of a skincare device, demand and supply of a skincare product in a cartridge form mounted on the skincare device, diagnosis of a skin condition necessary for skincare, and integrated management of skincare information provided by an expert and linked to the skincare product are possible.

In addition, a market size of a cartridge-replaceable skincare device capable of mixing and using different contents stored in independent cartridges by a predetermined amount may be expanded.

Effects obtainable by the skincare management platform and method based on a cartridge-replaceable skincare device according to the technical spirit of the present disclosure are not limited to the effects mentioned above, and other effects not mentioned will be clearly understood by a person having ordinary skill in the art to which the present disclosure pertains from the following description.

### [Description of Drawings]

FIG. 1 is an exemplary view of a skincare management platform based on a cartridge-replaceable skincare device according to an embodiment of the present disclosure.
FIG. 2 is an exemplary view of the scalp care device illustrated in FIG. 1.
FIG. 3 is an exemplary view of the face care device illustrated in FIG. 1.
FIG. 4 is a block diagram of the scalp care device of FIG. 2 and the face care device of FIG. 3.
FIG. 5 is a block diagram of the skin condition diagnosis apparatus illustrated in FIG. 1.
FIG. 6 is a flowchart of a skincare management method based on a cartridge-replaceable skincare device according to an embodiment of the present disclosure.

### [Best Mode for Carrying Out the Invention]

Before describing the present disclosure in detail, it should be understood that terms or words used in the present specification should not be unconditionally interpreted as being limited to ordinary or dictionary meanings, and the inventor of the present disclosure may appropriately define and use concepts of various terms in order to describe the inventor's invention in the best way, and further, these terms or words should be interpreted as meanings and concepts conforming to the technical spirit of the present disclosure.

That is, it should be understood that terms used in the present specification are only used to describe preferred embodiments of the present disclosure and are not used with an intention to specifically limit the content of the present disclosure, and these terms are terms defined in consideration of various possibilities of the present disclosure.

In addition, in the present specification, it should be understood that a singular expression may include a plural expression unless the context clearly indicates otherwise, and similarly, even when expressed in the plural, a singular meaning may be included.

Throughout the present specification, when a certain component is described as "including" another component, this may mean that any other component may be further included rather than excluding any other component, unless there is a description having a particularly opposite meaning.

Furthermore, when a certain component is described as "being present inside or connected to and installed in" another component, it should be understood that this component may be directly connected to or in contact with and installed in another component, or may be installed while being spaced apart by a certain distance, and in a case of being installed while being spaced apart by a certain distance, a third component or means for fixing or connecting the corresponding component to another component may be present, and a description of this third component or means may be omitted.

On the other hand, when a certain component is described as being "directly connected" or "directly coupled" to another component, it should be understood that a third component or means is not present.

Similarly, other expressions describing relationships between components, that is, "between" and "directly between," or "adjacent to" and "directly adjacent to," should also be interpreted as having the same purport.

In addition, in the present specification, terms such as "one surface," "the other surface," "one side," "the other side," "first," and "second," if used, are used so that one component can be clearly distinguished from another component with respect to the one component, and it should be understood that a meaning of the corresponding component is not limited by such terms.

In addition, in the present specification, terms related to positions such as "upper," "lower," "left," and "right," if used, should be understood as indicating relative positions in the corresponding drawing with respect to the corresponding component, and unless absolute positions are specified with respect to these positions, these position-related terms should not be understood as referring to absolute positions.

In addition, in the present specification, when reference numerals are added to components in each drawing, the same reference numerals indicate the same components throughout the specification so that the same components have the same reference numerals even when these components are shown in different drawings.

In the drawings attached to the present specification, sizes, positions, coupling relationships, and the like of components included in the present disclosure may be partially exaggerated, reduced, or omitted and described in order to sufficiently clearly convey the spirit of the present disclosure or for convenience of description, and therefore proportions or scales may not be strict.

In addition, in the following description of the present disclosure, detailed descriptions of configurations, for example, known technologies including conventional technologies, which are determined to unnecessarily obscure the gist of the present disclosure, may be omitted.

Hereinafter, embodiments of the present disclosure will be described in detail with reference to related drawings.

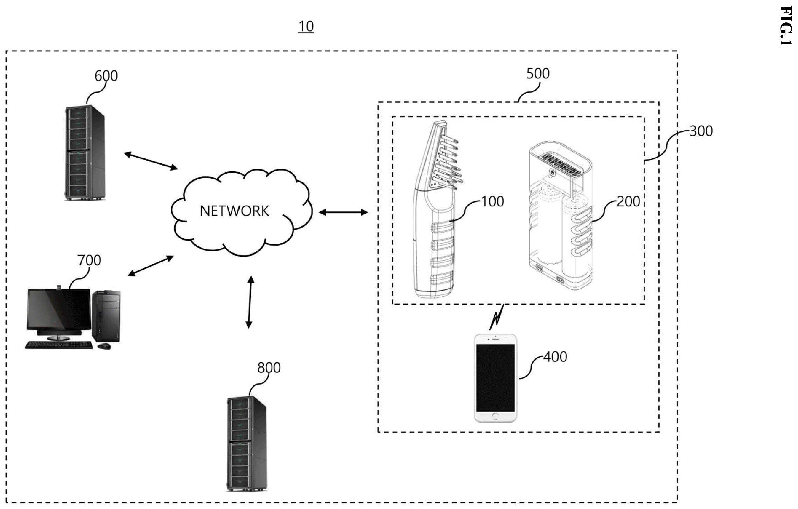

FIG. 1 is an exemplary view of a skincare management platform based on a cartridge-replaceable skincare device according to an embodiment of the present disclosure.

Referring to FIG. 1, a skincare management platform 10 based on a cartridge-replaceable skincare device according to an embodiment of the present disclosure (hereinafter, skincare management platform) is illustrated. The skincare management platform 10 may include a user system 500, a skin condition diagnosis apparatus 600, an expert terminal 700, and a maker terminal 800. The user system 500, the skin condition diagnosis apparatus 600, the expert terminal 700, and the maker terminal 800 may communicate with each other through a network, and may transmit and receive various information and data.

The user system 500 may include a skincare device 300 and a user terminal 400. The skincare device 300 is a device necessary for skincare, in which a consumable content is mounted in a cartridge form, and may include, for example, a scalp care device 100 and a face care device 200. The scalp care device 100 is a device that may be used for hair dyeing and hair care. The face care device 200 corresponds to a device capable of additionally performing various massages in order to spray cosmetics on a face, spread the cosmetics, and allow the cosmetics to permeate into skin.

The user terminal 400 is a communication terminal of the user and may include a so-called smartphone, tablet computer, and the like. The user terminal 400 may be connected through a network so as to communicate with the skincare device 300 by the Internet or short-range communication.

The skin condition diagnosis apparatus 600 may be implemented in a computer form, may be connected to a network and capable of diagnosing a skin condition of the user by using skin condition information, and may generate skin condition diagnosis information as a result.

The expert terminal 700 may provide a solution or prescription for skincare by using the skin condition diagnosis information generated by the skin condition diagnosis apparatus 600, and may generate skincare information including skincare product information as a result.

The maker terminal 800 may provide a customized skincare product selected according to a selection of the user who has received the skincare information to the user, or may provide other skincare information to the user.

When using the skincare device 300, the user may receive a diagnosis of the user's skin type and scalp condition. As a diagnosis method, the user terminal 400 collects skin condition information by using a camera, and the skin condition information is transmitted to the skin condition diagnosis apparatus 600. The skin condition diagnosis apparatus 600 collects data regarding a skin condition by using the skin condition information, diagnoses the skin condition by using the data regarding the skin condition, and transmits skin condition diagnosis information generated as a result to the expert terminal 700. When the expert terminal 700 transmits skincare information necessary for skincare of the user based on the skin condition diagnosis information, for example, skincare information regarding a skincare product, to the skin condition diagnosis apparatus 600, the skin condition diagnosis apparatus 600 transmits the received skincare information to the user terminal 400. The user selects a skincare product included in the skincare information through the user terminal 400, selected product information is transmitted to the maker terminal 800, and the maker terminal 800 may deliver to the user the skincare product selected by the user, that is, a product mountable on the skincare device 300 in a cartridge form. Therefore, the user communicates only with the skin condition diagnosis apparatus 600 through the user terminal 400, and does not need to be communicatively connected to the expert terminal 700 and the maker terminal 800. The skin condition diagnosis apparatus 600 has a function of mediating between the user terminal 400 and the expert terminal 700 and between the user terminal 400 and the maker terminal 800.

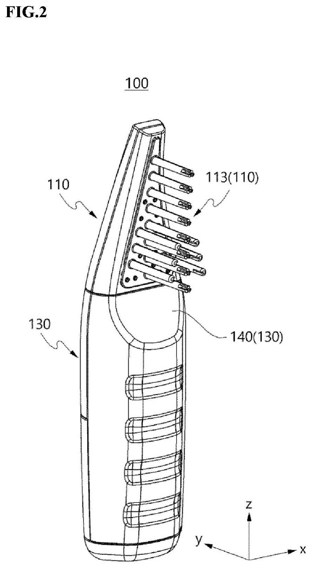

FIG. 2 is an exemplary view of the scalp care device illustrated in FIG. 1.

Referring to FIG. 2, the scalp care device 100 is illustrated. The scalp care device 100 may include a head 110, a comb 113 formed to protrude from the head 110, a body 130 coupled to the head 110, and a housing 140 surrounding a main body of the scalp care device 100 and having a picture formed on a surface thereof.

The body 130 includes a plurality of cartridges, for example, two cartridges of a first cartridge and a second cartridge, and different contents contained in the respective cartridges may be primarily discharged inside, mixed, and then secondarily discharged to the outside, that is, through a nozzle formed in the comb 113. Hereinafter, the face care device 200 in which the first cartridge and the second cartridge are illustrated will be described as an example.

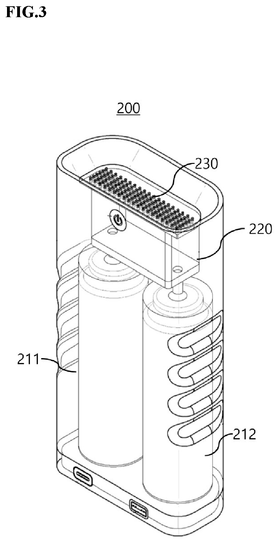

FIG. 3 is an exemplary view of the face care device illustrated in FIG. 1.

Referring to FIG. 3, the face care device 200 is illustrated. If the scalp care device 100 illustrated in FIG. 2 is a device used to care for a scalp or hair, the face care device 200 corresponds to a device used to care for skin other than the scalp, that is, skin of the whole body including a face. The face care device 200 illustrated in FIG. 3 corresponds to a device that sprays sunscreen in a mist form. A first content in a liquid form may be contained in a first cartridge 211 of the face care device 200, and a second content in a solid agent form may be contained in a second cartridge 212. When a discharge button is pressed, a mixer 220 sprays predetermined amounts of the first content and the second content inside by using ultrasonic waves to make a mixture, and the mixture may be sprayed through a discharge unit 230.

The skincare device 300 including the scalp care device 100 and the face care device 200 will be described through a block diagram as follows.

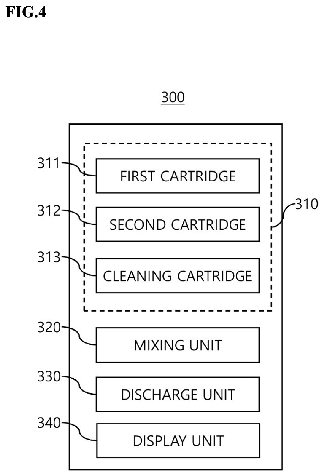

FIG. 4 is a block diagram of the scalp care device of FIG. 2 and the face care device of FIG. 3.

Referring to FIG. 4, the skincare device 300 may include a first cartridge 311 for storing a first content, a second cartridge 312 for storing a second content, a cleaning cartridge 313 replaceably mountable with the first cartridge 311 or the second cartridge 312, a mixing unit 320, a discharge unit 330, and a display unit 340.

The first cartridge 311 has a function of storing the first content and then internally discharging a predetermined amount of the first content to the mixing unit 320 by an action such as pressure.

The second cartridge 312 has a function of storing the second content and then internally discharging a predetermined amount of the second content to the mixing unit 320 by an action such as pressure.

The first content and the second content may form a hair dyeing agent and an oxidizing agent forming a hair dye, may form a first liquid agent and a second liquid agent corresponding to materials of a scalp care product or a face care product, or may form a solid agent and a liquid agent corresponding to materials of a scalp care product or a face care product in the same manner. Here, any one of the first liquid agent and the second liquid agent, or any one of the solid agent and the liquid agent may include a vitamin component. The vitamin component has a characteristic in which, when mixed with another component, an effect of the vitamin is reduced due to a chemical action, and therefore mixing immediately before use may maximize the effect of the vitamin. Therefore, cosmetics containing a vitamin component are divided into first content and second content and contained in cartridges, and a predetermined amount may be mixed before use, so that the effect of the vitamin component may be increased.

The cleaning cartridge 313 is used to remove residues of the first content of the first cartridge or the second content of the second cartridge when the residues remain in the mixing unit 320 or the discharge unit 330. That is, when the cleaning cartridge 313 is mounted at a position where the first cartridge 311 or the second cartridge 312 is mounted and driving for discharging a cleaning liquid contained in the cleaning cartridge 313 is performed, residues of the contents in the mixing unit 320 and the discharge unit 330 may be cleaned by the cleaning liquid.

The mixing unit 320 has a function of generating a mixture by mixing predetermined partial amounts of the first content and the second content with each other. In contrast to a conventional technology in which all of the first content and all of the second content are mixed at one time, a characteristic of the present disclosure is that an amount corresponding to a one-time use amount is internally discharged, mixed, and externally discharged.

The discharge unit 330 has a function of discharging the mixture through a nozzle formed in the discharge unit 330. The discharge unit 330 has a function of externally discharging the mixture through the nozzle by using pressure of the mixture.

The display unit 340 has a function of displaying remaining amount information of the first content and the second content and replacement information of the first cartridge 311 and the second cartridge 312. This alarm function may also be performed by the user terminal 400 capable of communicating with the skincare device 300. The skincare device 300 may interwork with the user terminal 400, collect history information regarding discharge of the mixture, collect cleaning history information regarding cleaning of the mixing unit and the discharge unit, and provide the same to the user.

Therefore, when the skincare device 300 combines discharge history information and cleaning history information, a time at which cleaning is necessary due to residues of the contents accumulated inside the device may be known. When cleaning of the mixing unit 320 and the discharge unit 330 is necessary, the skincare device 300 may display a message requesting cleaning to the user through the display unit 340. This cleaning request message may also be delivered to the user through the user terminal 400.

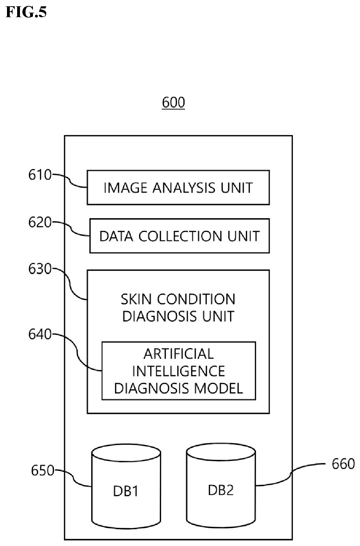

FIG. 5 is a block diagram of the skin condition diagnosis apparatus illustrated in FIG. 1.

Referring to FIG. 5, the skin condition diagnosis apparatus 600 may include an image analysis unit 610, a data collection unit 620, and a skin condition diagnosis unit 630. The skin condition diagnosis unit 630 may include an artificial intelligence diagnosis model 640.

The image analysis unit 610 of the skin condition diagnosis apparatus 600 has a function of analyzing an image included in skin condition information received from the user terminal 400 of the user system 500 by using a preset algorithm.

The data collection unit 620 has a function of collecting data regarding a skin condition by using an image analysis result of the image analysis unit 610.

The skin condition diagnosis unit 630 has a function of diagnosing a skin condition, for example, a degree of hair loss, by using data regarding the skin condition. The following is a mathematical equation into which the data regarding the skin condition is input.$\begin{matrix}DoHL = α \left(\frac{{N}_{avr} - N}{{N}_{avr}}\times 100\right) + β \left(\frac{{T}_{avr} - T}{{T}_{avr}}\times 100\right) + γ \left(\frac{{NH}_{avr} - NH}{{NH}_{avr}}\times 100\right) \\ + δ \left(\frac{EH - {EH}_{avr}}{EH}\times 100\right)\end{matrix}$

Information regarding data on the skin condition, that is, letters included in Equation 1, is as follows.
*Nₐᵥᵣ*: average number of hairs per unit area (for n normal persons)
*Tₐᵥᵣ*: average hair thickness (m hairs measured for n normal persons)
*NHₐᵥᵣ*: average number of hairs per follicle per unit area (for n normal persons)
*EHₐᵥᵣ*: average number of empty follicles per unit area (for n normal persons)
*N*: average number of hairs per unit area of a measurement subject
*T*: average hair thickness of the measurement subject (n hairs measured)
*NH:* average number of hairs per follicle per unit area of the measurement subject
*EH:* average number of empty follicles per unit area of the measurement subject
*α*: hair number constant (value calculated by the company)
*β*: hair thickness constant (value calculated by the company)
*γ*: average hair number per follicle constant (value calculated by the company)
*δ*: empty follicle number constant (value calculated by the company)

The skin condition diagnosis unit 630 of the skin condition diagnosis apparatus 600 may determine hair loss information of the user by using the data regarding the skin condition and Equation 1. DoHL described by Equation 1 is an index indicating the degree of hair loss, and indicates that the degree of hair loss is higher as the index approaches 1.

In addition, the skin condition diagnosis apparatus may include an artificial intelligence diagnosis model configured to diagnose a skin condition of the user by using skin condition information based on an ability to diagnose a skin condition acquired by learning, and generate the skin condition diagnosis information based on a result of the diagnosis.

The artificial intelligence diagnosis model is based on an artificial neural network, and may output data regarding a skin condition through learning of a previously prepared dataset, that is, skin condition information, for example, various images obtained by photographing a scalp or the like, or may determine a degree of hair loss corresponding to a result obtained by inputting the data regarding the skin condition into Equation 1.

In addition, the skin condition diagnosis apparatus may further include a first database DB1 650 storing scalp condition information according to at least one condition among age, sex, time, environmental condition, skin tone, hair length, disease, occupation, and scalp care history, and a second database DB2 660 storing degrees of scalp condition change of a plurality of users diagnosed and prescribed based on information in the first database DB1 650.

The skin condition diagnosis unit may diagnose a condition of the user's scalp and hair based on the skin condition information, and diagnose the user's scalp condition by comparing information and data stored in the first database 650 and the second database 660 with the skin condition information received from the user system 500. For example, the first database 650 includes initial information and data before the user receives skincare, that is, scalp care, and the second database 660 includes information and data collected after the user receives scalp care, so that the skin condition diagnosis unit 630 may diagnose the skin condition while comparing the initial data with data acquired thereafter.

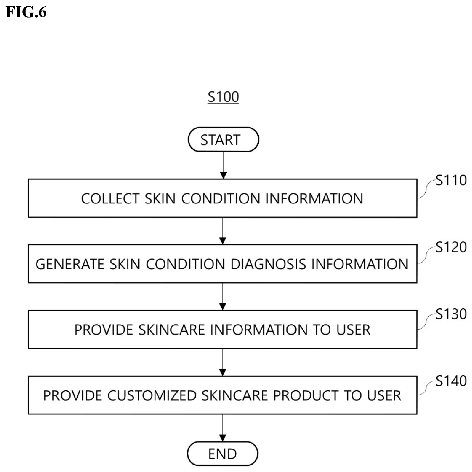

FIG. 6 is a flowchart of a skincare management method based on a cartridge-replaceable skincare device according to an embodiment of the present disclosure.

Referring to FIG. 6, a skincare management method S100 based on a cartridge-replaceable skincare device according to an embodiment of the present disclosure (hereinafter, skincare management method) is a method performed by the skincare management platform 10, and may include a step S110 of collecting skin condition information, a step S120 of generating skin condition diagnosis information, a step S130 of providing skincare information to the user, and a step S140 of providing a customized skincare product to the user.

The user system 500 included in the skincare management platform 10 may include the skincare device 300 on which a skincare product replaceable in a cartridge form is mountable, and may collect skin condition information regarding a skin condition of the user through a user terminal of the user using the skincare device 300 (S110).

The skin condition diagnosis apparatus 600 included in the skincare management platform 10 may receive the skin condition information from the user system, diagnose a skin condition of the user based on the skin condition information, and generate skin condition diagnosis information regarding the skin condition of the user according to a result of the diagnosis (S120).

The expert terminal included in the skincare management platform 10 may verify the skin condition diagnosis information and provide skincare information necessary for skincare of the user based on the skin condition diagnosis information (S130). The expert terminal 700 means a communication terminal of an expert capable of providing medical, pharmaceutical, and technical advice regarding the skin condition diagnosis information, for example, an expert such as a doctor, pharmacist, or engineer.

The maker terminal 800 included in the skincare management platform 10 may provide product information regarding a skincare product and a customized skincare product to the user based on the skincare information (S140). The maker terminal 800 means a communication terminal of a maker that manufactures and sells a skincare product, that is, a skincare product in a cartridge form.

For example, the user transmits image data obtained by photographing the user's scalp by using a camera included in the user terminal 400 to the skin condition diagnosis apparatus 600, and the skin condition diagnosis apparatus 600 analyzes the image data, extracts data regarding a skin condition, and outputs skin condition diagnosis information while determining a degree of hair loss by using Equation 1. The expert terminal 700 receives the skin condition diagnosis information and outputs skincare information based on the skin condition diagnosis information, that is, skincare information regarding a product necessary for scalp care, components included in the product, and the like, and the skincare information is delivered to the user terminal 400 through the skin condition diagnosis apparatus 600. The user selects a product suitable for the user based on the skincare information, selection information is transmitted to the maker terminal 800 through the skin condition diagnosis apparatus 600, and an order for the corresponding product is made. When the maker terminal 800 receives information regarding the skincare product from the skin condition diagnosis apparatus 600, the maker delivers the corresponding product to the user.

The skincare management platform 10 according to an embodiment of the present disclosure is characterized by being a platform in which the user system 500, the skin condition diagnosis apparatus 600, the expert terminal 700, and the maker terminal 800 all participate so that, when the user uses the skincare device 300, the user may receive diagnosis regarding a facial skin condition and a scalp condition through technology such as artificial intelligence, receive expert advice, and purchase a skincare product according to the advice, rather than merely individually using various skincare devices 300.

As described above, according to an embodiment of the present disclosure, in addition to demand and supply of a skincare device, demand and supply of a skincare product in a cartridge form mounted on the skincare device, diagnosis of a skin condition necessary for skincare, and integrated management of skincare information provided by an expert and linked to the skincare product are possible.

In addition, a market size of a cartridge-replaceable skincare device capable of mixing and using different contents stored in independent cartridges by a predetermined amount may be expanded.

Although various preferred embodiments of the present disclosure have been described above with some examples, descriptions of various embodiments described in the "Best Mode for Carrying Out the Invention" section are merely exemplary, and a person having ordinary skill in the art to which the present disclosure pertains will well understand that the present disclosure may be variously modified and implemented or embodiments equivalent to the present disclosure may be carried out from the above description.

In addition, since the present disclosure may be implemented in various other forms, the present disclosure is not limited by the above description, and the above description is only provided to make the disclosure of the present disclosure complete and to fully inform a person having ordinary skill in the art to which the present disclosure pertains of the scope of the present disclosure, and it should be understood that the present disclosure is defined only by each claim of the claims.

### [Industrial Applicability]

The present disclosure may be used in a field of manufacturing a skincare device and a skincare platform.

## Claims

1. A skincare management platform comprising:
a user system including a skincare device on which a skincare product replaceable in a cartridge form is mountable, and a user terminal configured to collect skin condition information regarding a skin condition of a user using the skincare device;
a skin condition diagnosis apparatus configured to receive the skin condition information from the user system, diagnose a skin condition of the user based on the skin condition information, generate skin condition diagnosis information regarding the skin condition of the user according to a result of the diagnosis, and provide information regarding a skincare product to the user based on skincare information; an expert terminal configured to verify the skin condition diagnosis information and provide the skincare information necessary for skincare of the user based on the skin condition diagnosis information; and
a maker terminal configured to provide a customized skincare product to the user based on the skincare information and a selection of the user.

2. The skincare management platform of claim 1, wherein the skincare device comprises at least one of:
a scalp care device on which a hair dye or scalp care product replaceable in a cartridge form is mountable; and
a face care device on which a face care product replaceable in a cartridge form is mountable.

3. The skincare management platform of claim 2, wherein the skincare device comprises:
a first cartridge containing a first content and a second cartridge containing a second content;
a mixing unit configured to generate a mixture by mixing predetermined partial amounts of the first content and the second content with each other; and
a discharge unit configured to discharge the mixture through a nozzle.

4. The skincare management platform of claim 3, wherein the first content and the second content comprise:
a hair dyeing agent and an oxidizing agent forming the hair dye;
a first liquid agent and a second liquid agent forming the scalp care product or the face care product; or
a solid agent and a liquid agent forming the scalp care product or the face care product,
wherein any one of the first liquid agent and the second liquid agent, or any one of the solid agent and the liquid agent comprises a vitamin component.

5. The skincare management platform of claim 3, wherein the skincare device further comprises a display unit capable of displaying:
remaining amount information of the first content and the second content; and
replacement information of the first cartridge and the second cartridge.

6. The skincare management platform of claim 5, wherein the display unit is configured to provide cleaning history information regarding cleaning of the mixing unit and the discharge unit to the user, and display a message requesting cleaning to the user when cleaning of the mixing unit and the discharge unit is necessary.

7. The skincare management platform of claim 6, wherein the skincare device further comprises a cleaning cartridge replaceable with the first cartridge and the second cartridge, and operates in a cleaning mode in which residues of the first content or the second content accumulated in the mixing unit and the discharge unit are removed by using the cleaning cartridge.

8. The skincare management platform of claim 1, wherein the skin condition diagnosis apparatus comprises: an image analysis unit configured to analyze an image included in the skin condition information received from the user system; a data collection unit configured to collect data regarding a skin condition by using a result of image analysis of the image analysis unit; and a skin condition diagnosis unit configured to diagnose a degree of hair loss by using the data regarding the skin condition.

9. The skincare management platform of claim 8, wherein the skin condition diagnosis apparatus is configured to determine a degree of hair loss of the user by using the data regarding the skin condition and the following equation: $\begin{matrix}DoHL = α \left(\frac{{N}_{avr} - N}{{N}_{avr}}\times 100\right) + β \left(\frac{{T}_{avr} - T}{{T}_{avr}}\times 100\right) + γ \left(\frac{{NH}_{avr} - NH}{{NH}_{avr}}\times 100\right) \\ + δ \left(\frac{EH - {EH}_{avr}}{EH}\times 100\right)\end{matrix}$
*Nₐᵥᵣ*: average number of hairs per unit area (for n normal persons)
*Tₐᵥᵣ*: average hair thickness (m hairs measured for n normal persons)
*NHₐᵥᵣ*: average number of hairs per follicle per unit area (for n normal persons)
*EHₐᵥᵣ*: average number of empty follicles per unit area (for n normal persons)
*N*: average number of hairs per unit area of a measurement subject
*T*: average hair thickness of the measurement subject (n hairs measured)
*NH:* average number of hairs per follicle per unit area of the measurement subject
*EH*: average number of empty follicles per unit area of the measurement subject
*α*: hair number constant (value calculated by the company)
*β*: hair thickness constant (value calculated by the company)
*γ*: average hair number per follicle constant (value calculated by the company)
*δ*: empty follicle number constant (value calculated by the company).

10. The skincare management platform of claim 1, wherein the skin condition diagnosis apparatus comprises an artificial intelligence diagnosis model configured to diagnose a skin condition of the user by using the skin condition information based on an ability to diagnose a skin condition acquired by learning, and generate the skin condition diagnosis information based on a result of the diagnosis.

11. The skincare management platform of claim 8, wherein the skin condition diagnosis apparatus further comprises:
a first database storing scalp condition information according to at least one condition among age, sex, time, environmental condition, skin tone, hair length, disease, occupation, and scalp care history; and
a second database storing degrees of scalp condition change of a plurality of users diagnosed and prescribed based on information in the first database,
wherein the skin condition diagnosis unit is configured to diagnose a condition of the user's scalp and hair based on the skin condition information, and diagnose the user's scalp condition by comparing information and data stored in the first database and the second database with the skin condition information received from the user system.

12. A skincare management method performed by a skincare management platform, the skincare management method comprising:
collecting, by a user system included in the skincare management platform, skin condition information regarding a skin condition of a user using a skincare device on which a skincare product replaceable in a cartridge form is mountable;
receiving the skin condition information from the user system, diagnosing a skin condition of the user based on the skin condition information, and generating skin condition diagnosis information regarding the skin condition of the user according to a result of the diagnosis;
verifying the skin condition diagnosis information and providing skincare information necessary for skincare of the user based on the skin condition diagnosis information; and
providing product information regarding a skincare product and a customized skincare product to the user based on the skincare information.
